# EUROPEAN PATENT APPLICATION

(11) **EP 4 137 074 A1**
(43) Date of publication of application: **22.02.2023**
(21) Application number: 21803771.1
(22) Date of filing: 08.05.2021
(51) Int. Cl.: A61B 17/34, A61B 34/20

(54) **PUNCTURE NEEDLE ASSEMBLY AND PUNCTURE SYSTEM**

(30) Priority: 12.05.2020 CN 202010399181
(71) Applicant: Shanghai Microport Ep Medtech Co., Ltd., Shanghai 201318 (CN)
(72) Inventor: ZHA, Feixiang, Shanghai 201318 (CN); LIANG, Bo, Shanghai 201318 (CN); SUN, Yiyong, Shanghai 201318 (CN); SONG, Yuwen, Shanghai 201318 (CN); LIU, Mengyao, Shanghai 201318 (CN)
(74) Representative: Marks & Clerk LLP
(86) International application number: PCT/CN2021/092514
(87) International publication number: WO 2021/228003

(57) **Abstract**

A puncture needle assembly (1000) and a puncture system (100) are provided. The puncture needle assembly (1000) includes a needle holder (1100) and a needle (1200). The needle holder (1100) comprises a channel axially extending therethrough. The channel has a sidewall, at least a part of which is provided by a conductor (1110). The needle (1200) has a proximal end portion extending in the channel and electrically connected to the conductor (1110). The puncture system (100) includes the puncture needle assembly (1000) and a dilator (2000). The dilator (2000) includes a dilation catheter (2100) and an optional conductive biasing member (2200) disposed on the dilation catheter (2100). Even when a distal end of the needle (1200) is hidden in the dilation catheter (2100), the conductive biasing member (2200) can still establish an electrical connection between the needle and the blood outside thereof. The puncture needle assembly (1000) and the puncture system (100) have the advantages of ease of use and high reliability.

## Description

### TECHNICAL FIELD

The present invention relates to the field of medical devices and, in particular, to a puncture needle assembly and a puncture system.

### BACKGROUND

Characterized by high morbidity, disability and mortality rates, cardiovascular disease poses a serious threat to human health. Interventional treatment is an effective therapeutic modality for cardiovascular disease. Interventional procedures for treating, for example, atrial fibrillation and atrial flutter typically involve puncturing the atrial septum to enable access of an interventional instrument to a target lesion site.

For transseptal puncture, a dilator and a puncture needle are indispensible. The puncture needle includes a distal stainless steel needle with puncturing capabilities which enable the dilator and a catheter sheath both fitted on the puncture needle to cross the atrial septum. A distal tapered portion of the dilator establishes a transition between the catheter sheath and the puncture needle, which can reduce resistance and avoid the creation of a large wound during the transseptal puncture and can shorten the puncturing process.

Traditionally, transseptal puncture is performed under the guidance of X-ray fluoroscopy, which is a two-dimensional visualization technique. In this context, a physician has to search for a target puncture site in a patient's body based on X-ray images and his/her manipulation. This practice is imprecise and risky, and sufficient dexterity of manipulation would require long-term clinical practice experience. In recent years, "green" electrophysiological three-dimensional locating systems have been widely used in the field of interventional procedures thanks to their advantages including extremely low radiation exposure and precise locating and navigation capabilities. They can greatly reduce the required training time, lower radiation exposure of both the physician and patient and enable more precise, safer and easier interventional procedures. However, these systems have seldom been used in transseptal puncture, and the two-dimensional modality relying on X-ray images and dexterous manipulation and lacking the advantages including extremely low radiation exposure and precise locating and navigation capabilities is still being employed. Therefore, at present, there is no precise puncture system operating under the guidance of both an electrophysiological locating system and electrocardiogram signals.

### SUMMARY OF THE INVENTION

It is an object of the present invention to provide a puncture needle assembly and a puncture system, which are usable in three-dimensional transseptal puncture, easy to use and reliable.

The above object is attained by a puncture needle assembly provided in the present invention, which comprises a needle holder and a needle. The needle holder comprises a channel axially extending therethrough. The channel has a sidewall, at least part of which is provided by a conductor. The needle has a proximal end portion disposed in the channel and electrically connected to the conductor.

Optionally, the conductor may comprise a block-shaped structure.

Optionally, an entire sidewall of the channel may be provided by the conductor.

Optionally, a part of the sidewall of the channel may be provided by the conductor, while a rest of the sidewall of the channel may be provided by an insulator.

Optionally, the insulator may comprise a first lumen axially extending therethrough and forming a part of the channel, wherein the insulator is provided with a window, wherein the window communicates with the first lumen and the conductor is disposed therein, and wherein an inner surface of the conductor and an inner surface of the first lumen form the channel.

Optionally, the insulator and the conductor may be arranged side by side in an axial direction of the needle holder and be coupled to one another, wherein the channel extends through each of the insulator and the conductor.

Optionally, the needle may be connected to the insulator.

Optionally, an inner surface of the conductor may be provided with an accommodating recess, wherein the needle holder comprises a first biasing element disposed in the accommodating recess, wherein the first biasing element is configured to store a biasing energy to cause the first biasing element to abut against the needle and thereby keep the needle be in an electrical connection with the conductor.

Optionally, the first biasing element may be a spring having an axis oriented perpendicular to an axis of the needle.

Optionally, the conductor may be connected to the needle via a conductive adhesive layer.

Optionally, the conductor may be provided with a socket for insertion of a contact pin of a connecting lead of an external locating device.

Optionally, the conductor may comprise a main body and an extension coupled to the main body, wherein the main body is coupled to the needle, with the socket being provided in the extension, wherein the needle holder further comprises an insulator comprising a first insulator portion and a second insulator portion, and wherein the extension is fitted over at least a part of the first insulator portion, with the main body being disposed between the first insulator portion and the second insulator portion.

The above object is attained by a puncture system provided in the present invention, which comprises a dilator and the puncture needle assembly as defined in any of the preceding paragraphs. The dilator comprises a dilation catheter, wherein the dilation catheter comprises a second lumen axially extending through the dilation catheter, and wherein a distal end of the needle is movably inserted into the second lumen.

Optionally, a side hole in communication with the second lumen may be provided in an inner surface of the dilation catheter at a distal end thereof.

Optionally, the dilator may further comprise a conductive biasing member disposed in the side hole so as to contact and to abut against the needle by a biasing force.

Optionally, the conductive biasing member may comprise a second biasing element comprising an arc-shaped portion, wherein the arc-shaped portion has a first end secured at the side hole, and a second end that is free, wherein the arc-shaped portion at least partially bulges beyond the inner surface of the dilation catheter.

Optionally, the conductive biasing member may further comprise an end cap and a frame, wherein the end cap is disposed in the side hole, wherein the frame is coupled to the end cap and is located between the end cap and the second lumen, and wherein the frame is coupled to the arc-shaped portion.

Optionally, the conductive biasing member may further comprise a stopper disposed on the arc-shaped portion, wherein when the needle comes into contact with the arc-shaped portion, the stopper is brought into contact with the end cap.

Optionally, an annular groove may be formed in an inner wall of the side hole, wherein the end cap comprises a protrusion that is complementary in shape to and engages with the annular groove.

The puncture needle assembly and puncture system of the present invention have the following advantages:
First, the puncture needle assembly includes a needle holder and a needle. The needle holder includes a conductor and comprises a channel axially extending therethrough. At least a part of a sidewall of the channel is provided by the conductor. A proximal end portion of the needle is disposed in the channel and is electrically connected to the conductor. During transseptal puncture, the conductor is coupled to a three-dimensional locating device to establish signal communication between the needle and the three-dimensional locating device. In this way, the puncture needle can be used in the field of surgical procedures aided by electrophysiological three-dimensional locating systems.

Second, the puncture needle assembly may further include a biasing member and/or a conductive adhesive layer, which can strengthen the connection between the needle and the conductor, and ensure desirable transmission of electrical signals during a puncture procedure, resulting in a high rate of success of the procedure.

Third, the conductor may be provided with a socket for insertion therein of a contact pin of the three-dimensional locating device, thereby achieving the advantages of ease of use and reliable connection.

Fourth, the puncture system includes the puncture needle assembly and a dilator. The dilator includes a dilation catheter comprising a second lumen axially extending therethrough. A side hole may be provided at a distal end of the dilation catheter so as to be in communication with the second lumen, thereby establishing electrical connection of the needle with the blood outside thereof. Optionally, the dilator may further include a conductive biasing member disposed in the side hole. During a transseptal puncture procedure, the needle may be advanced in the second lumen so that its distal end reaches the side hole and comes into contact with the conductive biasing member, resulting in the establishment of electrical connection between the needle and the blood outside thereof. That is, even when the distal end of the needle is hidden in the dilator, the conductive biasing member can still bring it into electrical connection with the blood to enable its location to be displayed on the three-dimensional system in real time while preventing it from causing injury to human tissue.

### BRIEF DESCRIPTION OF THE DRAWINGS

Fig. 1 is a schematic diagram showing the structure of a puncture system according to an embodiment of the present invention.
Fig. 2 schematically illustrates how transseptal puncture is performed by a puncture system according to an embodiment of the present invention.
Fig. 3 is a schematic enlarged view of a portion of the puncture system according to an embodiment of the present invention.
Fig. 4 is a cross-sectional view of the puncture system of Fig. 3 taken along A-A.
Fig. 5 is a schematic illustration of a portion of a puncture needle assembly in the puncture system according to an embodiment of the present invention.
Fig. 6 is a cross-sectional view of a portion of the puncture needle assembly in the puncture system according to an embodiment of the present invention, showing a conductor disposed in a window defined in an insulator.
Fig. 7 is a schematic illustration of a portion of the puncture needle assembly in the puncture system according to an embodiment of the present invention taken along a direction.
Fig. 8 is a schematic illustration of a portion of the puncture needle assembly in the puncture system of Fig. 7 taken along another direction, showing a socket provided in the conductor.
Fig. 9 is a cross-sectional view of the puncture needle assembly in the puncture system of Fig. 8 taken along B-B.
Fig. 10 is a cross-sectional view of a portion of a dilator in the puncture system according to an embodiment of the present invention.
Fig. 11 is a schematic diagram showing the structure of a conductive biasing member in the dilator of the puncture system of Fig. 10.
Fig. 12 is a cross-sectional view of a portion of the dilator in the puncture system according to another embodiment of the present invention.
Fig. 13 is a schematic diagram showing the structure of a conductive biasing member in the dilator of the puncture system of Fig. 12.
Fig. 14 is a schematic illustration of a distal end portion of a dilation catheter in the dilator of the puncture system according to an embodiment of the present invention, showing a side hole that has a shape of circle.
Fig. 15 is a cross-sectional view of the dilator in the puncture system of Fig. 14 taken along C-C.
Fig. 16 is a schematic illustration of the distal end portion of the dilation catheter in the dilator of the puncture system according to an embodiment of the present invention, showing a side hole that has a shape of a square.
Fig. 17 is a cross-sectional view of the dilator in the puncture system of Fig. 16 taken along D-D.

Description of Reference Numerals in the Drawings:
100 - Puncture System; 1000 - Puncture Needle Assembly; 1100 - Needle Holder; 1110 - Conductor; 1111 - Main Body; 1112 - Extension; 1113 - Socket; 1120 - Insulator; 1130 - First Biasing Element; 1200 - Needle; 2000 - Dilator; 2100 - Dilation Catheter; 2110 - Step; 2120 - Side Hole; 2200 - Conductive Biasing Member; 2210 - Second Biasing Element; 2211 - Arc-shaped Portion; 2220 - End Cap; 2221 - Protrusion; 2230 - Frame; 2240 - Stopper; 1131 - Accommodating Recess;
200 - Three-dimensional Locating Device;
300 - Catheter Sheath.

### DETAILED DESCRIPTION

Objects, advantages and features of the present invention will become more apparent from the detailed description set forth below when taken in conjunction with the accompanying drawings. Note that the figures are provided in a very simplified form not necessarily drawn to exact scale for the only purpose of facilitating easy and clear description of the disclosed embodiments.

As used herein, the singular forms "a", "an" and "the" include plural referents and the term "plurality" means two or more, unless the context clearly dictates otherwise. As used herein, the term "or" is generally employed in the sense of "and/or", unless the context clearly dictates otherwise. The terms "mounting", "coupling", "connecting", "joining" and grammatical variants thereof should be interpreted in a broad sense. For example, a connection may be a permanent, detachable or integral connection, or a mechanical or electrical connection, or a direct or indirect connection with one or more intervening media, or an internal communication or interaction between two components. Those of ordinary skill in the art can understand the specific meanings of the above-mentioned terms herein, depending on their context. Like numerals indicate like elements throughout the accompanying drawings.

As used herein, the terms "proximal" and "distal" are employed to describe relative orientations, relative positions and directions between components of a medical device or actions thereof, as viewed by a physician operating the device. Although not wishing to be limiting, a "proximal end" usually refers to an end closer to the physician, and a "distal end" usually refers to an end that first enters the body of a patient, during normal operation of the medical device.

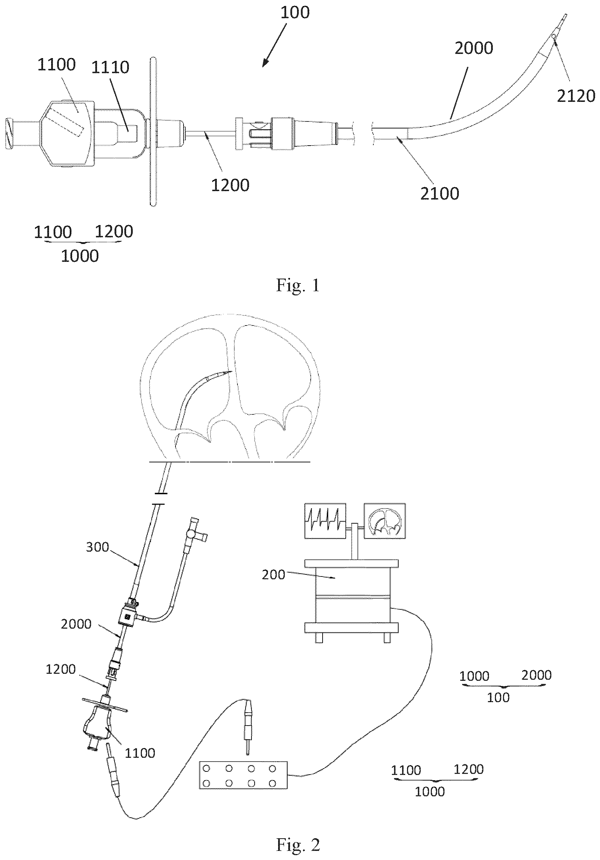

In embodiments of the present invention, there is provided a puncture needle assembly and a puncture system including the puncture needle assembly. Using the puncture system, a physician can perform puncture with the aid of a three-dimensional visual interface. As shown in Fig. 1, the puncture system 100 includes the puncture needle assembly 1000 and a dilator 2000. The puncture needle assembly 1000 includes a needle holder 1100 and a needle 1200. The needle holder 1100 includes a conductor 1110. The needle holder 1100 comprises a channel axially extending therethrough. At least a part of a sidewall of the channel is provided by the conductor 1110. A proximal end portion of the needle 1200 extends in the channel and is connected to the conductor 1110. The dilator 2000 includes a hollow dilation catheter 2100, and a distal end of the needle 1200 extends through the dilation catheter 2100 into human tissue to perform puncture.

As shown in Fig. 2, during puncture, the distal end of the needle 1200 establishes electrical connection with the blood for capturing electrical signal therefrom. The proximal end of the needle 1200 is coupled to a three-dimensional locating device 200 such as an electrophysiological locating device. The three-dimensional locating device 200 receives the electrical signals and, based thereon, locates the distal end of the needle 1200. In this embodiment, the conductor 1110 provided in the needle holder 1100 is coupled to the three-dimensional locating device, thereby establishing electrical connection between the needle 1200 and the three-dimensional locating device 200. This dispenses with the need to provide an additional lead on the needle 1100, enhancing manipulability of the puncture needle assembly 1000.

With combined reference to Fig. 1, in embodiments of the present invention, the conductor 1110 may be fabricated from a metallic conductive material such as stainless steel, aluminum or the like. The needle 1200 may be an elongate hollow structure made of a metallic conductive material. It may be either an integral one-piece tube or consisting of two tube sections that are coupled together. A distal end portion of the needle 1200 may be bendable, and the needle 1200 may have a diameter that is smaller at the distal end than at the proximal end. The distal end portion may be provided with a sharp tip which can facilitate puncture of human tissue. The proximal end portion of the needle 1200 is electrically connected to the conductor 1110, enabling transfer of the electrical signals to the conductor 1110 and further to the three-dimensional locating device 200.

In some embodiments, the needle holder 1100 is overall configured as the conductor 1110. In these cases, the entire sidewall of the channel is provided by the conductor 1110. For conventional puncture needles, physicians would want their needle holders to provide desired hand sensation during manipulation. This requires the needle holders to weigh in a certain range. Providing the needle holder overall as the conductor 1110 can satisfy this requirement. However, if the conductor is made of a metal, it will be expensive. Moreover, the commonly used metal copper has poor biocompatibility and must be subject to surface coating. However, as the channel is very narrow, its surface coating would be technically challenging. If biocompatible stainless steel is to be used as the material of the conductor, it would be difficult to machine it to a desired shape due to the poor plasticity.

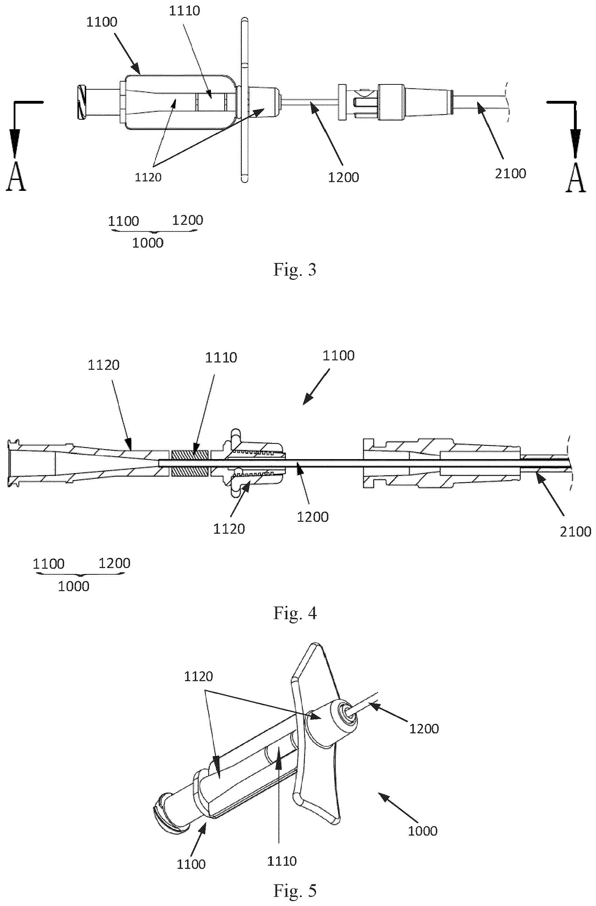

Therefore, in other embodiments, as shown in Fig. 3, the needle holder 1100 further includes an insulator 1120. In these cases, the insulator 1120 and the conductor 1110 may be separately fabricated and then coupled together. In this case, the conductor 1110 may provide a part of the sidewall of the channel, and the insulator 1120 may provide the rest of the sidewall of the channel. This can not only reduce the metallic material used and hence the cost of the puncture needle assembly 1000, but also allows the insulator 1120 to be made of a polymer material with good biocompatibility, which is safe for the human body and easy to process.

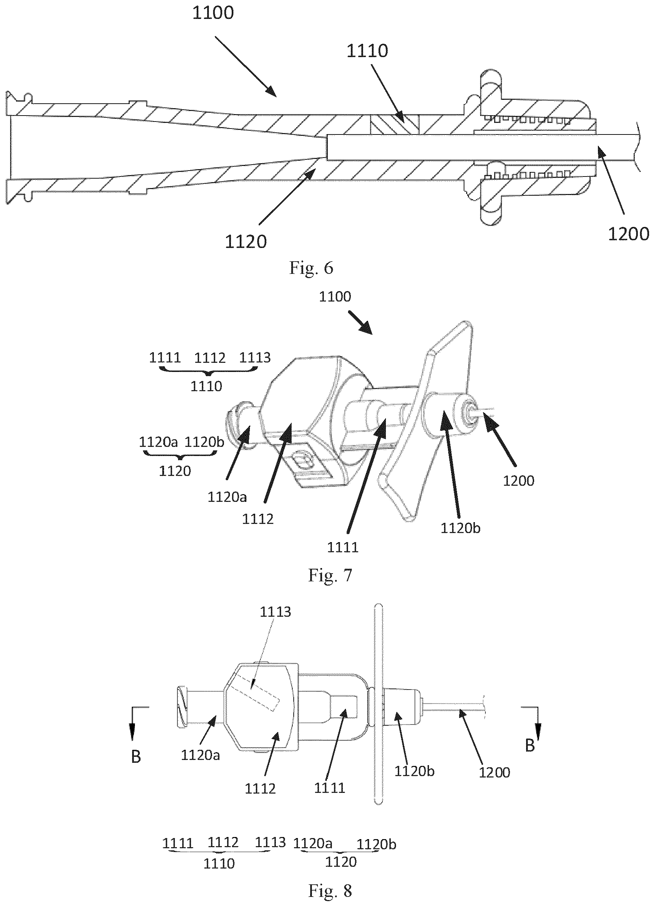

The structure of the needle holder 1100 may be designed as practically desired. However, the conductor 1110 is usually designed as a block-shaped structure. This design can not only facilitate gripping by the physician, but also enables the needle holder to have a desired weight that gives the physician desired hand sensation. Referring to Figs. 3 to 5, in one embodiment, the conductor 1110 and the insulator 1120 are axially arranged side by side in an axial direction of the needle holder 1100. The conductor 1110 comprises a first through bore that extends axially and the insulator comprises a second through bore that extends axially and communicates with the first through bore to make up the channel. Alternatively, as shown in Fig. 6, in another embodiment, the insulator 1120 comprises a first lumen axially extending therethrough and forming a part of the channel, and the insulator 1120 is provided with a window in communication with the first lumen. Moreover, the conductor 1110 is located within the window, and inner surface of the conductor 1110 and inner surface of the first lumen together define the channel. Generally, the needle 1200 is disposed within the channel so as to be connected to the conductor 1110. In order to facilitate assembly, an inner diameter of the channel is typically greater than an outer diameter of the proximal end portion of the needle 1200. However, this leaves a clearance between the needle 1200 and the conductor 1110, which makes their connection unreliable. In order to overcome this problem, in this embodiment, a conductive adhesive is filled between the inner surface of the conductor 1110 and an outer surface of the needle 1200. The conductive adhesive can cure into a conductive adhesive layer (not shown), which can strengthen the connection between the conductor 1110 and the needle 1200, ensuring smooth transmission of the electrical signals captured at the distal end of the needle 1200 to the three-dimensional locating device 200 via the conductor 1110.

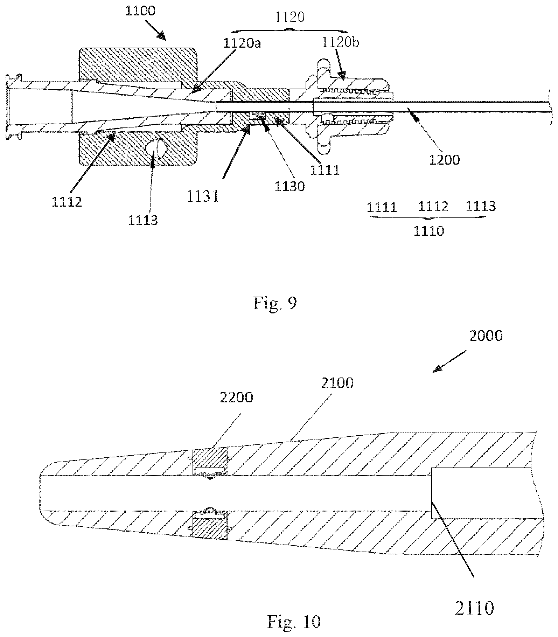

In another alternative embodiment shown in Fig. 9, a first biasing element 1130 may be provided to solve the problem of unreliable connection between the conductor 1110 and the needle 1200 that arises from the unmatched inner and outer diameters of the channel and the needle 1200. Specifically, an accommodating recess 1131 may be provided in the inner surface of the conductor 1110 (see Fig. 9), and the first biasing element 1130 may be disposed in the accommodating recess 1131 and configured to store biasing energy which causes the first biasing element 1130 to abut against the needle 1200, thereby bringing the needle 1200 into tight contact with the inner surface of the conductor 1110. Examples of the first biasing element 1130 may include, but are not limited to, a spring. In case of the first biasing element 1130 being implemented as a spring, an axis of the spring is perpendicular to an axis of the needle 1200.

The insulator 1120 may also be connected to the needle 1200 to enhance connection reliability of the needle 1200 with the needle holder 1100 and prevent dislodgement of the needle 1200 during use.

During a puncture process carried out by the puncture system 100 of this embodiment, the conductor 1110 in the puncture needle assembly 100 may be coupled to a connecting lead of the three-dimensional locating device 200 through a conductive clip.

However, when coupling the conductor 1110 to the connecting lead of the three-dimensional locating device 200 through a conductive clip, transmission of the electrical signals may be affected due to an insufficiently large electric contact area or unreliable electric contact. In view of this, in a modified embodiment, as shown in Figs. 7 to 9, the conductor 1110 is provide therein with a socket 1113 for receiving a contact pin of the connecting lead. This design can not only facilitate use, but is also advantageous in firm and reliable connection. Optionally, the conductor 1110 may include a main body 1111 and an extension 1112 joined to the main body 1111. The main body 1111 may be connected to the needle 1200, while the socket 1113 may be provided in the extension 1112. The main body 1111 may be axially connected to the needle 1200. The needle holder 1100 may include the insulator 1120 to reduce the metallic material used and hence the cost of the puncture needle assembly 1000 and enhance biological safety. The insulator 1120 may include a first insulator portion 1120a and a second insulator portion 1120b. The extension 1112 may be fitted over at least a part of the first insulator portion 1120a, and the main body 1111 may be provided between the first insulator portion 1120a and the second insulator portion 1120b. This design not only enables desired connection with the three-dimensional locating device 200 but also takes into account biological safety and cost. In the example with the contact pin mating with the socket 1113, all inner sidewalls of the socket 1113 may be conductive. This can additionally enhance the stability and reliability of signal transmission.

Based on the concept of the present invention, those skilled in the art can devise various variants of the needle holder, without departing from the scope of the invention. For example, the insulator may be partially fitted over the conductor, and the socket may be provided in the insulator. Moreover, the socket may extend to the conductor so that the contact pin, when inserted into the socket, can come into electrical connection with the conductor.

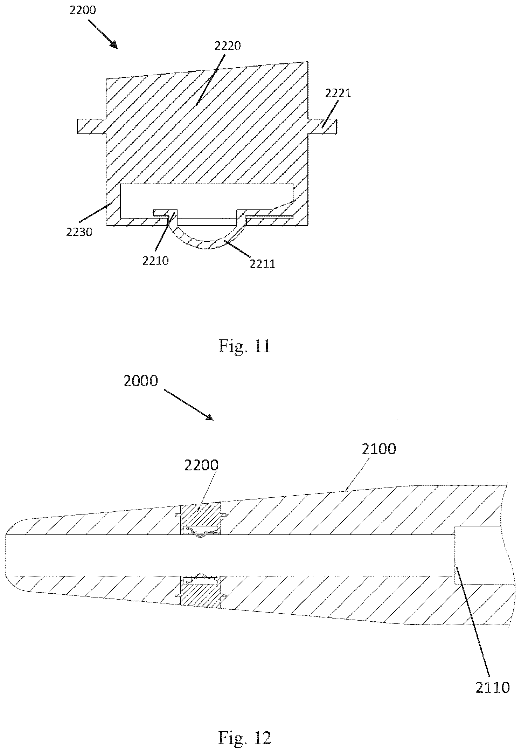

Referring to Fig. 1, in conjunction with Figs. 10 and 12, the dilation catheter 2100 of the dilator 2000 is an elongate structure and has a tip at distal end portion thereof. The dilation catheter 2100 comprises a second lumen extending axially therethrough, and an inner surface of the dilation catheter 2100 comprises a step 2110 for limiting a length of the needle 1200 that protrudes out of the distal end of the dilation catheter 2100 because an excessive protruded length of the needle 1200 may be hazardous.

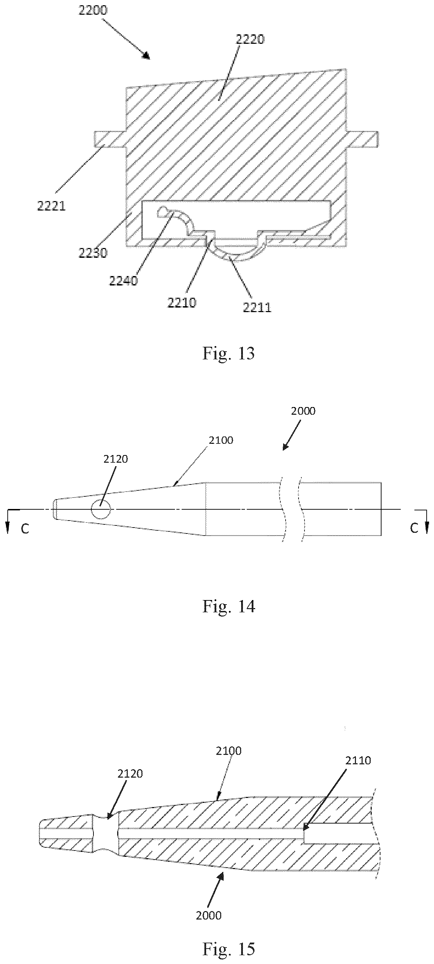

Further, referring to Figs. 14 to 17, the dilation catheter 2100 comprises, around the distal end thereof, a side hole 2120 in communication with the second lumen of the dilation catheter 2100. The side hole 2120 can establish electrical connection of the needle 1200 with the blood outside of the dilation catheter 2100. In this design, during a search for a target puncture site after a distal end of the puncture system 100 enters a patient's body, the distal end of the needle 1200 can be hidden within the dilation catheter 2100 of the dilator 2000 while capturing electrical signals by virtue of its electrical connection with the blood established by the side hole 2120, thus avoiding causing injury to human tissue during the search for the target puncture site.

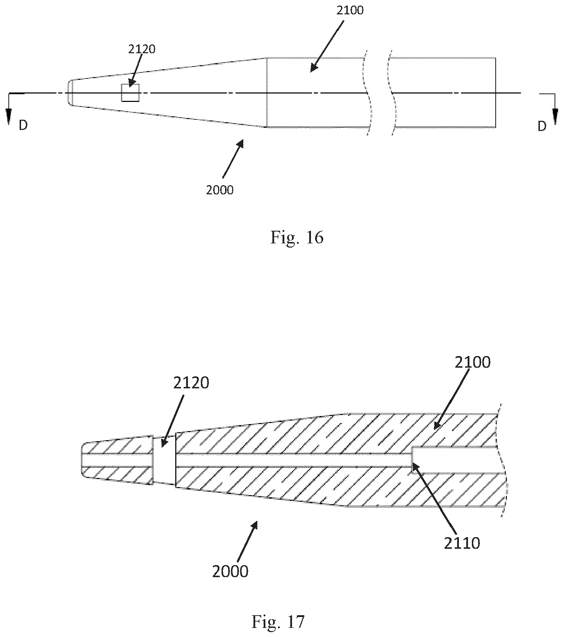

In this embodiment, the side hole 2120 may have any suitable cross-sectional shape, such as circular (see Figs. 14 and 15), square (see Figs. 16 and 17), oblong, polygonal, irregular, etc. Further, either one or more such side holes 2120 may be provided. For example, two side holes 2120 may be provided in symmetry at the distal end of the dilation catheter 2100.

In order to enhance the electrical connection between the needle 1200 and the blood, in another embodiment, the dilator 2000 further includes a conductive biasing member 2200, which is disposed within the side hole 2120. When the conductive biasing member 2200 comes into contact with the blood, electrical connection between the blood and the needle 1200 is established.

With continued reference to Figs. 10 and 11, in an exemplary embodiment, the conductive biasing member 2200 includes a second biasing element 2210, which is a curved metal sheet comprising an arc-shaped portion 2211. One end of the arc-shaped portion 2211 is secured at the side hole 2120, and the other end thereof is free. At least a part of the arc-shaped portion 2211 bulges beyond the inner surface of the dilation catheter 2100. During the search for the target puncture site, the arc-shaped portion 2211 abuts against the needle 1200 around the distal end thereof to bring the needle 1200 into effective contact with the conductive biasing member 2200.

Optionally, the conductive biasing member 2200 may further include an end cap 2220 and a frame 2230. The end cap 2220 may be integrally formed with the frame 2230, or they may be separate components which are joined together suitably, for example, by welding. The end cap 2220 may be secured to an inner sidewall of the side hole 2120, and the non-free end of the arc-shaped portion 2211 may be secured to an inner sidewall of the frame 2230. Additionally, as shown in Figs. 12 and 13, the conductive biasing member 2200 may further include a stopper 2240 disposed on the arc-shaped portion 2211. Preferably, the stopper 2240 is joined to the free end of the arc-shaped portion 2211. When the needle 1200 comes into contact with the arc-shaped portion 2211, the second biasing element 2210 will be deflected to cause the stopper 2240 to move toward the end cap 2220 and finally come into contact with the end cap 2220. In this way, the conductive biasing member 2200 can not only enhance the electrical connection between the needle 1200 and the blood but can also give the physician improved hand sensation. For example, upon the distal end of the needle 1200 coming into contact with the arc-shaped portion 2211, the needle 1200 will be subject to resistance from the arc-shaped portion 2211, providing the physician with tactile feedback that informs the physician in a timely manner of the arrival of the distal end of the needle 1200 at the side hole.

Furthermore, an annular groove may be formed in the inner sidewall of the side hole 2120, and the end cap 2220 may comprise a protrusion 2221 that is complementary in shape to the annular groove. The protrusion 2221 may engage with the annular groove, thereby firmly securing the end cap 2220 in the side hole 2120.

A transseptal puncture process performed by the puncture system 100 according to embodiments of the present invention will be described below with reference to Fig. 2. The transseptal puncture process includes the following steps:
Step S1: Advance a guidewire in the right femoral vein to the junction of the superior vena cava and the subclavian vein.

Step S2: Assemble the dilator 2000 with a catheter sheath 300, and advance them together over the guidewire into the superior vena cava.

Step S3: Withdraw the guidewire and flush the catheter sheath 300 with a heparin-saline solution.

Step S4: Couple the conductor 1110 of the puncture needle assembly 1000 to the three-dimensional locating device 200 and insert the needle 1200 into the dilation catheter 2100 of the dilator 2000 until the distal end of the needle 1200 reaches the side hole 2120 and comes into contact with the conductive biasing member 2200, thereby enabling the three-dimensional locating device 200 to locate the distal end of the needle 1200.

Step S5: Retract the catheter sheath 300, the dilator 2000 and the needle 1200, rotate the needle 1200, and determine the fossa ovalis based on the locations of the His bundle and coronary sinus in an image displayed on the three-dimensional locating device 200, and stop retracting the catheter sheath 300, the dilator 2000 and the needle 1200 upon determination of the fossa ovalis.

Step S6: Identify the target puncture site which is at a location of the septum 1/3 posterior to the His bundle level.

Step S7: Advance the dilator 2000 and the catheter sheath 300 a distance of about 2-3 mm.

Step S8: With the catheter sheath 300 and the dilator 2000 being held stationary, advance the needle 1200 about 5-10 mm according to an image displayed on the three-dimensional locating device 200 so that the distal end of the needle 1200 extends out of the distal end of the dilation catheter 2100 and penetrates the atrial septum.

Step S9: With the needle 1200 being held stationary, advance the catheter sheath 300 and the dilator 2000 so that a distal end of the dilator 2000 enters the left atrium.

Step S 10: Withdraw the needle 1200 out of the patient's body, with the dilator 2000 still being held stationary.

Step S11: Advance the catheter sheath 300 so that its distal end also enters the left atrium.

Step S12: With the catheter sheath 300 being held stationary, withdraw the dilator 2000 out of the patient's body, thus ending the puncture process.

Embodiments of the present invention provide a puncture needle assembly and a puncture system. The puncture needle assembly includes a needle holder and a needle. The needle holder includes a conductor, and a proximal end portion of the needle is disposed in the needle holder and connected to the conductor. The conductor establishes signal communication between the needle and a three-dimensional locating device, which can increase safety and a success rate of transseptal puncture. The puncture system includes the puncture needle assembly and a dilator. The dilator includes a dilation catheter comprising a lumen axially extending therethrough. The dilation catheter is distally provided therein with a side hole in communication with the lumen, and a conductive biasing member is disposed in the side hole. During a search for a target puncture site, the needle is hidden within the dilation catheter, with electrical connection with the blood outside of the dilation catheter being established by the conductive biasing member, which enables the three-dimensional locating device to locate the needle. In this way, higher safety can be achieved by avoiding causing injury to the superior vena cava or endocardial tissue.

Although the present invention has been disclosed hereinabove, it is not limited to the above disclosure. Those skilled in the art can make various changes and modifications to the invention without departing from the spirit and scope thereof. Accordingly, it is intended that any and all such changes and modifications also fall within the scope of the present invention as defined by the appended claims and equivalents thereof.

## Claims

1. A puncture needle assembly comprising a needle holder and a needle, wherein the needle holder comprises a channel axially extending therethrough, wherein the channel comprises a sidewall, and at least a part of the channel is provided by a conductor, and wherein the needle comprises a proximal end portion that extends in the channel and is electrically connected to the conductor.

2. The puncture needle assembly according to claim 1, wherein the conductor comprises a block-shaped structure.

3. The puncture needle assembly according to claim 1 or 2, wherein an entire sidewall of the channel is provided by the conductor.

4. The puncture needle assembly according to claim 1 or 2, wherein a part of the sidewall of the channel is provided by the conductor, while a rest of the sidewall of the channel is provided by an insulator.

5. The puncture needle assembly according to claim 4, wherein the insulator comprises a first lumen axially extending therethrough and forming a part of the channel, wherein the insulator is provided with a window, wherein the window communicating with the first lumen, and wherein the conductor is disposed therein, wherein an inner surface of the conductor and an inner surface of the first lumen form the channel.

6. The puncture needle assembly according to claim 4, wherein the insulator and the conductor are arranged side by side in an axial direction of the needle holder, are and coupled to one another, and wherein the channel extends through each of the insulator and the conductor.

7. The puncture needle assembly according to claim 4, wherein the needle is connected to the insulator.

8. The puncture needle assembly according to claim 1, wherein an inner surface of the conductor is provided with an accommodating recess, wherein the needle holder comprises a first biasing element disposed in the accommodating recess, wherein the first biasing element is configured to store a biasing energy to cause the first biasing element to abut against the needle and thereby keep the needle be in an electrical connection with the conductor.

9. The puncture needle assembly according to claim 8, wherein the first biasing element is a spring having an axis oriented perpendicular to an axis of the needle.

10. The puncture needle assembly according to claim 1 or 8, wherein the conductor is connected to the needle via a conductive adhesive layer.

11. The puncture needle assembly according to claim 1, wherein the conductor is provided with a socket for insertion of a contact pin of a connecting lead of an external locating device.

12. The puncture needle assembly according to claim 11, wherein the conductor comprises a main body and an extension coupled to the main body, wherein the main body is coupled to the needle, with the socket being provided in the extension, wherein the needle holder further comprises an insulator comprising a first insulator portion and a second insulator portion, and wherein the extension is fitted over at least a part of the first insulator portion, with the main body being disposed between the first insulator portion and the second insulator portion.

13. A puncture system comprising a dilator and the puncture needle assembly as defined in any one of claims 1 to 12, wherein the dilator comprises a dilation catheter, wherein the dilation catheter comprises a second lumen axially extending through the dilation catheter, and wherein a distal end of the needle is movably inserted into the second lumen.

14. The puncture system according to claim 13, wherein a side hole in communication with the second lumen is provided in an inner surface of the dilation catheter at a distal end thereof.

15. The puncture system according to claim 14, wherein the dilator further comprises a conductive biasing member disposed in the side hole so as to contact and to abut against the needle by a biasing force.

16. The puncture system according to claim 15, wherein the conductive biasing member comprises a second biasing element comprising an arc-shaped portion, wherein the arc-shaped portion has a first end secured at the side hole, and a second end that is free, and wherein the arc-shaped portion at least partially bulges beyond the inner surface of the dilation catheter.

17. The puncture system according to claim 16, wherein the conductive biasing member further comprises an end cap and a frame, wherein the end cap is disposed in the side hole, wherein the frame is coupled to the end cap and is located between the end cap and the second lumen, and wherein the frame is coupled to the arc-shaped portion.

18. The puncture system according to claim 17, wherein the conductive biasing member further comprises a stopper disposed on the arc-shaped portion, wherein when the needle comes into contact with the arc-shaped portion, the stopper is brought into contact with the end cap.

19. The puncture system according to claim 18, wherein an annular groove is formed in an inner wall of the side hole, wherein the end cap comprises a protrusion that is complementary in shape to and engages with the annular groove.
